# EUROPEAN PATENT APPLICATION

(11) **EP 1 779 870 A1**
(43) Date of publication of application: **02.05.2007**
(21) Application number: 06122839.1
(22) Date of filing: 24.10.2006
(51) Int. Cl.: A61L 9/04, A61L 9/12

(54) **Active substance controlled release device for sanitization of spaces susceptible of containing degenerating organic material**

(30) Priority: 26.10.2005 IT TO20050760
(71) Applicant: Marcopolo Engineering SPA, 12011 Borgo San Dalmazzo (IT)
(72) Inventor: Esposito, Elisabetta, 44100 Ferrara (IT); Cortesi, Rita, 44100 Ferrara (IT); Menegatti, Enea, 44100 Ferrara (IT); Mascalzi Gianni, 20045 Besana Brianza (Milano) (IT)
(74) Representative: Freyria Fava, Cristina

(57) **Abstract**

The present invention concerns a device for the controlled release of substances capable of controlling the release of malodorous gases in environments where organic matrices are present that may give rise to decomposition.

## Description

### TEXT OF THE DESCRIPTION

The present invention relates to an active substance controlled release device for the sanitization of spaces in which organic matrices are present that may decompose and give rise to malodorous gases.

To reduce the malodorous gases originating from the decomposition of the organic substances and eliminate the pathogenic germs present, for example, in refuse bins of the dumpster type for the collection of solid urban waste or in the litter of farmed animals, it is necessary to provide for the cleaning of such dumpsters or litter and for their sanitization, in a systematic manner. These operations are also intensified during the summer months, when the temperature increase favours decomposition reactions of organic waste and thus the proliferation of germs.

The problem of malodorous emissions should not be neglected, since it involves not only the population that lives in the proximity of the dumpster or farm, but also the health of the staff charged with operations of emptying and cleaning the dumpsters or the litter in poultry farms.

To limit the formation and emission of malodorous gases in dumpsters, different solutions may be adopted, such as for example frequent washing or treatment with associations of micro-organisms that contrast the formation of unpleasant odours. Normally, dumpsters are washed with hot water at high pressure or with steam at a high temperature at intervals of approximately one month.

At present, spraying or washing of dumpsters (where this is practised) is undertaken at the moment of emptying the dumpster. As soon as the dumpster has been emptied into the compactor, one or more nozzles spray the inside of the dumpster with detergent and degreasing products, sometimes combined with products having a bacterial or enzymatic base or with associations of moulds-fungi-bacteria that control the degradation of the organic matrix to be collected inside the dumpster.

In some cases, spraying after washing is manual; in other cases the product comprising a bacterial or enzymatic base or an association of fungi-moulds-bacteria is contained in a compartment in the cover of the dumpster from which the active substances may diffuse.

Several different products are known to technology that can be used to control the production of malodorous gases originating from organic substances subject to decomposition. An example is given by the patent document JP-A-2003-061652, which describes a controlled release preparation of micro-organisms that is capable of releasing the micro-organisms in a manner useful to control the degradation of organic materials. In this particular solution, the micro-organisms are selected from among lactobacilli, actinomycetes, photosynthetic bacteria, yeasts and filamentous bacteria; the matrix within which these micro-organisms are dispersed comprises a polymer that presents the capability of releasing the micro-organisms when it comes into contact with water.

The document JP-A-2002205938 also concerns a controlled release composition capable of releasing bacteria or substances useful to control the degradation of organic materials in a controlled manner. The solution described in the above Japanese document concerns beads/pellets based on gels containing bacteria or substances useful for the purpose, in particular alginic acid or derivatives of alginic acid, that are released into the environment of interest.

The United States patent US-A-4 511 552 describes a system to deodorise environments, in which the system may take three different forms: a gel to be applied onto the surfaces of the environment in question, a solid product that may take different forms (pellets, sticks) that can also be used in poultry farms, and a product capable of floating in the case in which a liquid surface is present in the environment to be deodorised. The active agents contained within these devices comprise cultures of bacteria, fungi and enzymes as well as nutrients for these organisms.

The present invention has as its purpose that of providing a device for the controlled and gradual release of active substances, in particular for dumpsters for the collection of solid urban waste, which is more efficacious compared to what has up to now been put into effect in the treatment and/or prevention of the formation of malodorous gases originating from the degradation of organic waste.

According to the present invention, this purpose is achieved thanks to a solution described specifically in the attached claims. The claims form an integral part of the technical instruction provided here in regard to the invention.

In an embodiment at present preferred, the present invention concerns a device for the controlled release of substances useful for the sanitization of environments in which organic matrices that may decompose are present, such as for example dumpsters, farmed animal litter, coffins, and deposits of organic materials in general. In particular, the purpose of the invention relates to two essential functions in the sphere of closed environments, in which biodegradation of organic substances persists:
1. to enable the micro-organisms present in the device to attack the organic substances present in the dumpster, malodorous and liable to rot, so as to control its decomposition with less malodorous forms;
2. to enable the micro-organisms present in the device to attack the volatile organic substance emitted by the primary degradation of the organic substances so as to limit the release of malodorous gases;
3. to enable the micro-organisms present in the device to attack the organic substance that becomes stratified on the inner surface of the dumpster every day between filling and emptying the dumpster, since the inner surface of the dumpster thus caked with degradable substances are the major cause of unpleasant odours since they are only washed occasionally and the inner walls remain caked among others also by organic substances that can easily degrade/putrefy with a high capability of producing malodorous gases, as well as providing a favourable nesting place for the proliferation of insects and of giving rise to possible viral and pathogenic agents;
4. in the case of coffins, it becomes a device providing full respect of the deceased, naturally at the discretion of the customer.

Among the substances useful for this purpose, as an example and without limiting intent, the following may be mentioned: associations of moulds-fungi-bacteria, micro-organisms, enzymes or chemical compounds having the capability of contrasting the formation of malodorous gases originating from processes of organic decomposition.

The invention will now be described in detail, as a simple example without limiting intent, with reference to the single attached figure, in which a possible embodiment of the device according to the present invention is illustrated.

A device according to the present invention presents numerous advantages with regard to what the technology has made available up to now. In particular, in the case in which it is desired to limit the formation of malodorous gases in dumpsters for the collection of solid urban waste, the device according to the present invention may be applied immediately after the dumpster has been washed by the compactor that empties the dumpster. Furthermore, application of the device does not depend on external factors connected with the compactor, such as for example the load of sanitising/disinfectant liquid, or the proper functioning of the spraying system.

Again, the procedure for the application of the present device can easily be standardised and may come about at the time of washing the dumpster.

Again, the device according to the present invention enables the active product to be released as a function of the conditions effectively existing inside the dumpster, for example in conditions of high temperature the release is accelerated, whereas in conditions in which the temperature is not such as to give rise to degradation phenomena, the release of these micro-organisms and active substances is slowed.

The device according to the present invention can also be "personalised" as a function of requirements or of environmental conditions or of the composition of the waste present inside the dumpster. The device according to the present invention, furthermore, presents no openings towards the outside and, for this reason, is protected against the entrance of insects and/or other animals that might damage the device.

Again, the device according to the present invention does not require an energy supply for its operation and does not decrease the loading capacity of the dumpster.

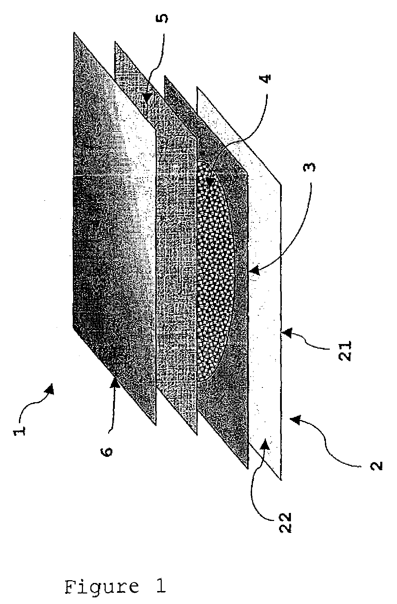

An embodiment of the device according to the present invention that is preferred at present is shown in figure 1.

The device, indicated overall with reference number 1, presents a general sandwich structure. It comprises a support structure 2, for example of polyester, polyethylene or aluminium, in the shape of a dish or plate, if desired fitted with an adhesive surface 21 useful to maintain the device 1 in place after it has been positioned inside the environment to be sanitised. On the non-adhesive surface 22 of the support structure 2, a substratum 3 is present on which a material 4 is positioned, containing the active substance or substances to be released. In direct contact with the material 4, a layer of technical fabric or membrane 5 is situated, with the function of protecting the material 4. A protective layer 6 is present above the technical layer or membrane 5, to be removed at the time of using the device 1.

The operating principle of the controlled release device according to the present invention is based on the capability of certain technical fabrics and/or membranes 5 to be permeable in a mono-directional manner; that is to release the micro-organisms or the active substances contained in the material in a single direction, for example in the case of application in a dumpster for the collection of waste, towards the inside of the dumpster and not towards the support structure 2.

At this point, as a function of the temperature and humidity conditions inside the dumpster, the device 1 will release a greater or a lesser quantity of active substances, thus optimising its action in terms of consumption over time. The quantity of active substance inside the material 4 is optimised as a function of the mean time between the application of a first device and the replacement of that device.

An example of an active substance that can be utilised to advantage in the sphere of the present invention consists of an association of natural moulds-fungi-bacteria, not genetically modified, such as for example the association contained in the commercial product ENZYVEBA, produced by the company MARCOPOLO.

The material 4 comprises a matrix within which the active substance or substances are dispersed, in which the matrix presents characteristics of gradual and/or controlled release of the active substance or substances. In an embodiment at present preferred, the material 4 presents a matrix consisting of structured materials capable of carrying and slowly releasing the active substance, such as for example the microbiological association ENZYVEBA.

These structured materials consist of gels based on mixtures of hydrophilic polymers and/or copolymers dispersed in the microbiological complex ENZYVEBA. The polymers and/or copolymers used, once they are dispersed, form a three-dimensional structured lattice (gel) that enables the ENZYVEBA to be released in a controlled manner towards the outside environment.

The appropriate selection of polymeric materials and their ratio by weight enables the speed of release of ENZYVEBA to be protracted for up to three weeks from the opening of the package containing the gel. The structured material maintains the microbiological activity of the microbiological complex unaltered.

As an example without limiting intent, the structured materials that can be employed to advantage in the sphere of the present invention include: mixtures of hydrophilic polymers and/or copolymers such as polyethylenes, polyoxyethylenes, polypropylenes and their copolymers, polyvinylchlorides, polymethacrylates, polyamides, polyacrylics (carbomers, modified carbomers, copolymers) polyalkylenes (polyvinylic alcohol, unsubstituted polyalkylenes, substituted copolymers), polyoxyalkylenes, polymers deriving from acetylene, from 1-vinylpyrrolidone, from alkylvinylethers, modified hydrophilic polysiloxanes. In particular, polymers compatible with the high bacterial load of the microbiological association to be used are employed.

Naturally, construction details and embodiments may be widely varied with respect to what is described and illustrated, without thereby departing from the sphere of the present invention, as defined by the attached claims.

## Claims

1. Device 1 for the controlled release of at least one active substance capable of being utilised to sanitise environments where degradable organic matrices are present, comprising:
-- a support structure 2,
-- a material 4 comprising a matrix containing at least one active substance positioned on the support structure 2,
-- a layer of technical fabric or membrane 5 situated in direct contact with the material 4.

2. Device according to claim 1, **characterised in that** it also includes a protective layer 6 applied onto the layer of technical fabric or membrane 5.

3. Device according to claim 1 or claim 2, **characterised in that** a substratum 3 is situated between the structure 2 and the material 4.

4. Device according to any of the above claims, **characterised in that** the structure 2 takes the form of a dish or of a plate.

5. Device according to any of the above claims, **characterised in that** the structure 2 is made of polyester, polyethylene or aluminium.

6. Device according to any of the above claims, **characterised in that** the layer of technical fabric or membrane 5 consists of a composite structural material.

7. Device according to any of the above claims, **characterised in that** the layer of technical fabric or membrane 5 preferably comprises polyester-polythene (PET-PE), paper or aluminium.

8. Device according to any of the above claims, **characterised in that** the matrix of material 4 substantially comprises a gel based on a mixture of hydrophilic polymers and/or copolymers capable of forming a structured three-dimensional lattice.

9. Device according to claim 8, **characterised in that** the polymers and/or copolymers are selected from among polyethylenes, polyoxyethylenes, polyoxypropylenes and their copolymers, polyvinylchlorides, polymethacrylates, polyamides, polyacrylics (carbomers, modified carbomers, copolymers) polyalkylenes (polyvinylic alcohol, unsubstituted polyalkylenes, substituted copolymers), polyoxyalkylenes, polymers deriving from acetylene, from 1-vinylpyrrolidone, from alkylvinylethers, modified hydrophilic polysiloxanes.

10. Device according to any of the above claims, **characterised in that** the active substance is selected from among associations of non-pathogenic and not genetically modified moulds-fungi-bacteria, micro-organisms, enzymes or chemical compounds having the capability of controlling the release of malodorous gases originating from processes of organic decomposition.

11. Device according to claim 10, **characterised in that** the micro-organisms contained in the material 4 are of natural origin.

12. Device according to claim 10, **characterised in that** the chemical compounds contained in the material 4 are non-toxic and are not deleterious.

13. Use of a controlled release device containing at least one active substance capable of controlling the release of malodorous gases for the sanitization of environments in which degradable organic matrices are present.

14. Use according to claim 13, **characterised in that** the active substance is selected from among associations of moulds-fungi-bacteria, micro-organisms, enzymes or chemical compounds having the capability of controlling the release of malodorous gases originating from processes of organic decomposition.

15. Use according to claim 13 or claim 14, **characterised in that** the micro-organisms, enzymes or chemical compounds are released as a function of the conditions of temperature and humidity in the environment in which said device is present.

16. Use according to any of the claims from 13 to 15, **characterised in that** the environments are selected from among dumpsters for the collection of solid urban waste, litter of farmed animals, deposits of organic materials, coffins.

17. Use according to any of the claims from 13 to 16, **characterised in that** the device 1 comprises:
-- a support structure 2,
-- a material 4 consisting of a matrix containing at least one active substance positioned on the support structure 2, and
-- a layer of technical fabric or membrane 5 situated in direct contact with the material 4.

18. Use according to claim 17, **characterised in that** the device 1 also includes a protective layer 6 applied onto the layer of technical fabric or membrane 5.

19. Device according to claim 17, **characterised in that** the device 1 also includes a substratum 3 positioned between the structure 2 and the material 4.

20. Use according to claim 17, **characterised in that** the structure 2 takes the form of a dish or plate.

21. Use according to claim 17, **characterised in that** the structure 2 consists of polyester, polyethylene or aluminium.

22. Use according to claim 17, **characterised in that** the layer of technical fabric or membrane 5 comprises a composite structural material.

23. Use according to claim 17, **characterised in that** the layer of technical fabric or matrix 5 preferably consists of polyester-polythene (PET-PE), paper or aluminium.

24. Use according to claim 17, **characterised in that** the matrix of material 4 substantially comprises a gel based on mixtures of hydrophilic polymers and/or copolymers capable of forming a structured three-dimensional grid.

25. Use according to claim 24, **characterised in that** the polymers and/or copolymers are selected from among polyethylenes, polyoxyethylenes, polypropylenes and their copolymers, polyvinylchlorides, polymethacrylates, polyamides, polyacrylics (carbomers, modified carbomers, copolymers) polyalkylenes (polyvinylic alcohol, unsubstituted polyalkylenes, substituted copolymers), polyoxyalkylenes, polymers deriving from acetylene, from 1-vinylpyrrolidone, from alkylvinylethers, modified hydrophilic polysiloxanes.
